# EUROPÄISCHE PATENTSCHRIFT

(11) **EP 2 625 157 B1**
(45) Veröffentlichungstag und Bekanntmachung des Hinweises auf die Patenterteilung: **31.05.2017**
(21) Anmeldenummer: 11764539.0
(22) Anmeldetag: 04.10.2011
(51) Int. Cl.: C07C 29/38, C07C 33/025

(54) **HERSTELLUNG VON HOMOALLYLALKOHOLEN IN ANWESENHEIT VON NICHT-KOVALENT IMMOBILISIERTEN IONISCHE FLÜSSIGKEITS - PHASE KATALYSATOREN UNTER GASPHASENREAKTIONSBEDINGUNGEN**
PREPARATION OF HOMOALLYL ALCOHOLS IN THE PRESENCE OF NONCOVALENTLY IMMOBILIZED IONIC LIQUID PHASE CATALYSTS UNDER GAS PHASE REACTION CONDITIONS
PRODUCTION D'ALCOOLS HOMOALLYLIQUES EN PRÉSENCE DE CATALYSEURS EN PHASE LIQUIDE IONIQUE IMMOBILISÉS NON COVALENTS DANS DES CONDITIONS DE RÉACTION EN PHASE GAZEUSE

(30) Priorität: 05.10.2010 EP 10186581
(43) Veröffentlichungstag der Anmeldung: 14.08.2013
(73) Patentinhaber: BASF SE, 67056 Ludwigshafen am Rhein (DE)
(72) Erfinder: STOCK, Christoph, 67158 Ellerstadt (DE); GERHARD, Dirk, 68163 Mannheim (DE); EBEL, Klaus, 68623 Lampertheim (DE)
(86) Internationale Anmeldenummer: PCT/EP2011/067244
(87) Internationale Veröffentlichungsnummer: WO 2012/045707

(56) Entgegenhaltungen:
- JYOTHI T M ET AL: "A LEWIS ACID CATALYST ANCHORED ON SILICA GRAFTED WITH QUATERNARY ALKYLAMMONIUM CHLORIDE MOIETIES", ANGEWANDTE CHEMIE. INTERNATIONAL EDITION, WILEY VCH VERLAG, WEINHEIM, Bd. 40, Nr. 15, 3. August 2001 (2001-08-03), Seiten 2881-2884, XP001075306, ISSN: 1433-7851, DOI: 10.1002/1521-3773(20010803)40:15<2881::AID -ANIE2881>3.0.CO;2-P
- DATABASE CA [Online] CHEMICAL ABSTRACTS SERVICE, COLUMBUS, OHIO, US; LI, XUEFENG ET AL: "Synthesis of 3-methylbut-3-en-1-ol over immobilized SnCl4 catalyst", XP002668447, gefunden im STN Database accession no. 2010:1115257 & LI, XUEFENG ET AL: "Synthesis of 3-methylbut-3-en-1-ol over immobilized SnCl4 catalyst", SHIYOU HUAGONG , 39(8), 909-912 CODEN: SHHUE8; ISSN: 1000-8144, 2010,
- VAN DOORSLAER ET AL: "Immobilization of molecular Catalysts in supported ionic liquids", DALTON TRANSACTIONS, Bd. 39, Nr. 36, 23. April 2010 (2010-04-23), Seiten 8377-8390, XP055018096, ISSN: 1477-9226, DOI: 10.1039/c001285H
- GAJANAN RASHINKAR ET AL: "An expeditious synthesis of homoallylic alcohols using Brønsted acidic supported ionic liquid phase catalyst with pendant ferrocenyl group", CATALYSIS COMMUNICATIONS, Bd. 12, Nr. 15, 1. September 2011 (2011-09-01), Seiten 1442-1447, XP55018064, ISSN: 1566-7367, DOI: 10.1016/j.catcom.2011.05.026

## Beschreibung

Die Erfindung betrifft die selektive Herstellung von Homoallylalkoholen aus Alkenen und Aldehyden oder Ketonen in Anwesenheit von nicht-kovalent immobilisierten Ionische Flüssigkeits - Phase Katalysatoren unter Gasphasenreaktionsbedingungen.

Homoallylalkohole können beispielsweise durch eine als Prins-Reaktion bekannte Umsetzung hergestellt werden, welche allgemein die elektrophile Addition eines Aldehyds oder Ketons an ein Alken bezeichnet, gefolgt von der Addition eines Nukleophils an das entstehende Intermediat. Auch eine En-Reaktion kommt hierfür in Frage, die allgemein als eine pericyclische Reaktion bekannt ist, bei der ein Alken, welches ein Wasserstoff in allylischer Position trägt, mit einer Verbindung, die eine Mehrfachbindung besitzt, umgesetzt wird. Sie verläuft schlechter, je höher der Substitutionsgrad in der allylischen Position, die das zu übertragende Proton trägt, ist.

Nachfolgend wird der Stand der Technik zusammengefasst:
JACS 1955, 77, 4666-8 beschreibt die unkatalysierte thermale Addition von Paraformaldehyd an Dialkyl-substituierte terminale Olefine unter erhöhtem Druck und erhöhter Temperatur. Im speziellen wird die Umsetzung von Isobuten zu 3-Methyl-3-buten-1-ol beschrieben, und die Einflüsse diverser Reaktionsbedingungen dargestellt.

Aus JACS 1982, 104, 555-63 ist die katalysierte Prins-Reaktion von Aldehyden bekannt. Als allgemein problematisch an Lewis-Säure katalysierten En-Reaktionen von Aldehyden (Paraformaldehyd) wird der gebildete Alkohol-Lewis-Säure Komplex dargestellt, der empfindlich gegen Solvolyse ist. Zudem ist sie eine starke protische Säure, welche die Doppelbindung protonieren kann.

In US 4,511,751 wird die Reaktion von Isobuten und / oder tertiärem Butanol mit Formaldehyd in einer sauer-wässrigen Lösung beschrieben. Weil die Alkoholfunktion eliminiert wird, ist das Reaktionsprodukt (bei Temperaturen > 150°C) nicht der ungesättigte Alkohol Isoprenol, sondern Isopren.

Chem. Commun. 2001, 992-3 lehrt, dass die direkte Immobilisierung von Katalysatoren auf anorganischen Trägermaterialien unvorteilhaft sein kann, weil es aufgrund von Auslaugungen zu Aktivitätsverlusten kommen kann. Alternativ wird vorgestellt, dass der Metallkatalysator (SnCl₄) über Tetraalkylammoniumchlorid am Träger (MCM-41) verankert werden kann und damit die Aktivität erhalten bleiben könnte. Das Kation wird über eine Siloxan-Funktionalität kovalent an die Oberfläche des Trägers gebunden, wobei diese chemische Fixierung mehrere chemische Reaktionen erforderlich macht. Umsetzungen von Isobuten mit Paraformaldehyd im nichtwässrigen Medium haben die Hypothese bestätigt.

Gemäß Angew. Chem. Int. Ed. 2005, 44, 815-9 können SILP (supported ionic liquid phase) Katalysator-Systeme für die Olefin-Hydroformylierung (Rh-katalysiert), Hydrogenierung (Rh-katalysiert), Heck-Reaktionen (Pd-katalysiert) und Hydroaminierung (Rh-, Pd- und Znkatalysiert) verwendet werden können. Die Übergangsmetallkomplexe sind dabei in einem dünnen Film von Ionischer Flüssigkeit gelöst, welcher an einem porösen Feststoff mit großer Oberfläche über Physisorption oder auch kovalente Verankerung gehalten wird.

In Angew. Chem. Int. Ed., 2001, 40, 2881-2884 sowie in der Chemical Abstracts Service Datenbank zu Shiyou Huagong, 2010, 39(8), 909-912 werden Verfahren zur Herstellung von Isoprenol aus Isobuten durch Prins-Reaktion mit Formaldehyd mittels eines auf einer Festphase immobilisierten Katalysators beschrieben.

Technische Aufgabe war die selektive Herstellung von Homoallylalkoholen durch Umsetzung von Alkenen mit Aldehyden oder Ketonen unter milden Reaktionsbedingungen, z.B. Normaldruck. Es galt einen dafür geeigneten Katalysator zu finden. Vorteilhafterweise soll eine solche Reaktion in Anwesenheit von Wasser stattfinden können, da häufig Formaldehyd als Ausgangsstoff verwendet wird, der wiederum in wässriger Form kostengünstig ist. Das Verfahren sollte vorteilhafterweise kontinuierlich durchgeführt werden können, was voraussetzt, dass die Katalysatoraktivität erhalten bleibt. Ferner ist eine hohe Raum-Zeit-Ausbeute erwünscht.

Gelöst wurde die Aufgabe durch ein Verfahren zur Herstellung von Homoallylalkoholen durch katalysierte Umsetzung von geradkettigen oder verzweigten C₃-C₆ Alkenen mit Aldehyden oder Ketonen, dadurch gekennzeichnet dass die Umsetzung in Gegenwart von nicht-kovalent immobilisierten Ionische Flüssigkeits - Phase Katalysatoren in der Gasphase erfolgt, wobei die nicht-kovalent immobilisierten Ionische Flüssigkeits - Phase Katalysatoren hydrophobe ionische Flüssigkeiten sind, die ausgewählt sind unter Imidazoliumsalzen, welche nicht-kovalent an einen Träger gebunden sind und welche wenigstens eine Lewis-Säure umfassen, wobei die Wasserlöslichkeit der hydrophoben ionischen Flüssigkeit bei 20°C und 1 bar weniger als 100 g Wasser in 1000 g ionischer Flüssigkeit beträgt. Vorteilhaft ist, dass der Katalysator aktiv erhalten bleibt und damit eine kontinuierliche Reaktionsführung möglich ist. Besonders vorteilhaft ist, dass das gefundene Herstellverfahren die Anwesenheit von Wasser erlaubt.

Überraschend war, dass durch Einsatz der SILP-Technologie (supported ionic liquid phase; hier "immobilisierte Ionische Flüssigkeits - Phase" genannt) eine hohe Selektivität bei der Umsetzung beispielsweise von Olefinen mit wässrigem Formaldehyd, zum Homoallylalkohol erzielt wird. Außerdem hat sich erwiesen, dass das immobilisierte Ionische Flüssigkeits - Phase System aktiv während der Reaktionsführung erhalten bleibt. Durch Umsetzung in der Gasphase kann im Überschuss eingesetztes, und nicht verbrauchtes Olefin wieder in die Reaktion rückgeführt werden. Die Reaktionsführung unter Normaldruck ist möglich.

Nachfolgend wird das zur Anwendung gekommene Katalysatorensystem beschrieben:
Unter dem Begriff Ionische Flüssigkeiten werden hier Salze (Verbindungen aus Kationen und Anionen) verstanden, die bei Normaldruck (1 bar) einen Schmelzpunkt < 350 °C, vorzugsweise < 300 °C, besonders bevorzugt < 250 °C und ganz bevorzugt < 100 °C besitzen. In einer besonders bevorzugten Ausführungsform sind die Ionischen Flüssigkeiten unter Normaldruck (1 bar) und bei Reaktionstemperatur flüssig.

Erfindungsgemäße ionische Flüssigkeiten sind ausgewählt sind unter Imidazoliumsalzen.

Bevorzugt sind Imidazolium-Kationen wie sie in der unten stehenden Formel I aufgeführt sind. 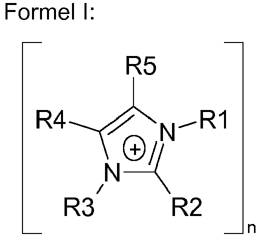 worin
R1 und R3 für einen unter Reaktionsbedingungen inerten organischen Rest mit 1 bis 20 C-Atomen stehen,
R2, R4, und R5 für ein H-Atom oder einen unter Reaktionsbedingungen inerten organischen Rest mit 1 bis 20 C-Atomen stehen,
und n für 1, 2 oder 3 steht.

In Formel I stehen R1 und R3 vorzugsweise unabhängig für einen unter Reaktionsbedingungen inerten organischen Rest mit 1 bis 10 C-Atomen. Insbesondere stehen R1 und R3 für einen aliphatischen, araliphatischen oder aromatischen Rest, insbesondere einen aliphatischen, araliphatischen oder aromatischen Rest ohne weitere Heteroatome, z. B. für eine Alkylgruppe. Besonders bevorzugt stehen R1 und R3 unabhängig voneinander für eine geradkettige oder verzweigte C₁-C₁₀ beispielsweise eine C₁-C₄ Alkylgruppe wie Methyl, Ethyl, Propyl, Butyl, iso-Butyl.

In Formel I stehen R2, R4 und R5 vorzugsweise unabhängig für ein H-Atom oder einen organischen Rest mit 1 bis 10 C-Atomen; insbesondere stehen R2, R4 und R5 für ein H-Atom oder einen aliphatischen, araliphatischen oder aromatischen Rest. Besonders bevorzugt stehen R2, R4 und R5 unabhängig voneinander für ein H-Atom oder eine Alkylgruppe, insbesondere stehen R2, R4 und R5 unabhängig voneinander für ein H-Atom oder eine geradkettige oder verzweigte C₁-C₄ Alkylgruppe wie Methyl, Ethyl, Propyl, Butyl, iso-Butyl. Ganz besonders bevorzugt stehen R2, R4 und R5 jeweils für ein H-Atom.

Die Ionischen Flüssigkeiten können anorganische oder organische Anionen enthalten. Derartige Anionen sind z.B. in WO 03/029329, WO 2007/076979, WO 2006/000197 und WO 2007/128268 aufgeführt.

Als Anion in Betracht kommen insbesondere solche aus
- der Gruppe der Halogenide und halogenhaltigen Verbindungen der Formeln: F⁻, Cl⁻, Br, I⁻, BF₄⁻, PF₆⁻, AlCl₄⁻, Al₂Cl₇⁻, Al₃Cl₁₀⁻, AlBr₄⁻, FeCl₄⁻, BCl₄⁻, SbF₆⁻, AsF₆⁻,⁻ZnCl₃⁻, SnCl₃⁻, CuCl₂⁻, CF₃SO₃⁻, C₄F₉SO₃⁻, C₈F₁₇SO₃⁻, (CF₃SO₃)₂N⁻, (C₂F₅SO₂)₂N⁻, CF₃CO₂⁻, CCl₃CO₂⁻, CN-, SCN-, OCN-, NO²⁻, NO³⁻, N(CN)-;
- der Gruppe der Sulfate, Sulfite und Sulfonate der allgemeinen Formeln: SO₄²⁻, HSO₄⁻, SO₃²⁻, HSO₃⁻, R^{a}OSO₃⁻, R^{a}SO₃⁻;
- der Gruppe der Phosphate der allgemeinen Formeln: PO₄³⁻, HPO₄²⁻, H₂PO₄⁻, R^{a}PO₄²⁻, HR^{a}PO₄⁻, R^{a}R^{b}PO₄⁻;
- der Gruppe der Phosphonate und Phosphinate der allgemeinen Formel: R^{a}HPO₃⁻, R^{a}R^{b}PO₂⁻⁻, R^{a}R^{b}PO₃⁻;
- der Gruppe der Phosphite der allgemeinen Formeln: PO₃³⁻, HPO₃²⁻, H₂PO₃⁻, R^{a}PO₃²⁻, R^{a}HPO₃⁻, R^{a}R^{b}PO₃⁻;
- der Gruppe der Phosphonite und Phosphinite der allgemeinen Formel: R^{a}R^{b}PO₂⁻, R^{a}HPO₂⁻, R^{a}R^{b}PO⁻, R^{a}HPO⁻;
- der Gruppe der Carboxylate der allgemeinen Formeln: R^{a}COO⁻;
- der Gruppe der Borate der allgemeinen Formeln: BO₃³⁻, HBO₃²⁻, H₂BO₃⁻, R^{a}R^{b}BO₃⁻, R^{a}HBO₃⁻, R^{a}BO₃²⁻, B(OR^{a})(OR^{b})(OR^{c})(OR^{d})⁻, B(HSO₄)⁻, B(R^{a}SO4)⁻;
- der Gruppe der Boronate der allgemeinen Formeln: R^{a}BO₂²⁻, R^{a}R^{b}BO⁻;
- der Gruppe der Carbonate und Kohlensäureester der allgemeinen Formeln: HCO₃⁻, CO₃²⁻, R^{a}CO₃⁻;
- der Gruppe der Silikate und Kieselsäuresäureester der allgemeinen Formeln: SiO₄⁴⁻, HSiO₄³⁻ H₂SiO₄²⁻, H₃SiO₄⁻, R^{a}SiO₄³⁻, R^{a}R^{b}SiO₄²⁻, R^{a}R^{b}R^{c}SiO₄⁻, HR^{a}SiO₄²⁻, H₂R^{a}SiO₄⁻, HR^{a}R^{b}SiO4⁻;
- der Gruppe der Alkyl- bzw. Arylsilan-Salze der allgemeinen Formeln: R^{a}SiO₃³⁻, R^{a}R^{b}SiO₂²⁻, R^{a}R^{b}R^{c}SiO⁻, R^{a}R^{b}R^{c}SiO₃⁻, R^{a}R^{b}R^{c}SiO₂⁻, R^{a}R^{b}SiO₃²⁻;
- der Gruppe der Carbonsäureimide, Bis(sulfonyl)imide und Sulfonylimide der allgemeinen Formeln: 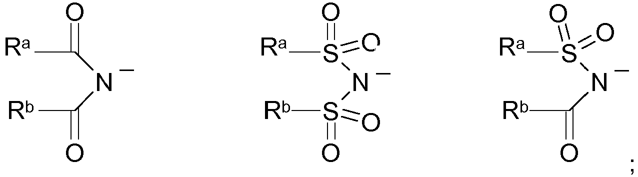
- der Gruppe der Methanide der allgemeinen Formel: 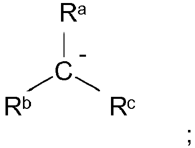
- der Gruppe der Halometallate der allgemeinen Formel: [MᵣHalₜ]^{s-},
   wobei M für ein Metall und Hal für Fluor, Chlor, Brom oder Iod steht, r und t ganze positive Zahlen sind und die Stöchiometrie des Komplexes angeben und s eine ganze positive Zahl ist und die Ladung des Komplexes angibt;
- der Gruppe der Sulfide, Hydrogensulfide, Polysulfide, Hydrogenpolysulfide und Thiolate der allgemeinen Formeln: S²⁻, HS-, [Sᵥ]²⁻, [HSᵥ]⁻, [R^{a}S]⁻,
   wobei v eine ganze positive Zahl von 2 bis 10 ist; und
- der Gruppe der komplexen Metallionen wie Fe(CN)₆³⁻, Fe(CN)₆⁴⁻, MnO₄⁻, Fe(CO)₄⁻.

In den vorstehenden Anionen bedeuten R^{a}, R^{b}, R^{c} und R^{d} unabhängig voneinander jeweils
- Wasserstoff;
- C₁-C₃₀ Alkyl und deren aryl-, heteroaryl-, cycloalkyl-, halogen-, hydroxy-, amino-, carboxy-, -O-, -CO-O- oder-CO-N< substituierte Komponenten, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosyl, Octacosyl, Nonacosyl, Triacontyl, Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl, 3-Cyclohexylpropyl, Methoxy, Ethoxy, Acetyl oder C_{q}F_{2(q-a)+(1-b)} H_{2a+b} mit q ≤ 30, 0 ≤ a ≤ q und b = 0 oder 1 (beispielsweise CF₃, C₂F₅, CH₂CH₂-C_{(q-2)}F_{2(q-2)+1}, C₆F₁₃, C₈F₁₇, C₁₀F₂₁, C₁₂F₂₅);
- C₃-C₁₂-Cycloalkyl und deren aryl-, heteroaryl-, cycloalkyl-, halogen-, hydroxy-, amino-, carboxy-, -O-, oder -CO-O- substituierte Komponenten, wie beispielsweise Cyclopentyl, 2-Methyl-1-cyclopentyl, 3-Methyl-1-cyclopentyl, Cyclohexyl, 2-Methyl-1-cyclohexyl, 3-Methyl-1-cyclohexyl, 4-Methyl-1-cyclohexyl oder C_{q}F_{2(q-a)-(1-b)} H_{2a-b} mit q ≤ 30, 0 ≤ a ≤ q und b = 0 oder 1;
- C₂-C₃₀-Alkenyl und deren aryl-, heteroaryl-, cycloalkyl-, halogen-, hydroxy-, amino-, carboxy-, -O-, oder -CO-O- substituierte Komponenten, wie beispielsweise 2-Propenyl, 3-Butenyl, cis-2-Butenyl, trans-2-Butenyl oder C_{q}F_{2(q-a)-(1-b)} H mit q ≤ 30, 0 ≤ a ≤ q und b = 0 oder 1;
- C₃-C₁₂-Cycloalkenyl und deren aryl-, heteroaryl-, cycloalkyl-, halogen-, hydroxy-, amino-, carboxy-, -O-, oder -CO-O- substituierte Komponenten, wie beispielsweise 3-Cyclopentenyl, 2-Cyclohexenyl, 3-Cyclohexenyl, 2,5-Cyclohexadienyl oder C_{q}F_{2(q-a)-3(1-b)} H_{2a-3b} mit q ≤ 30, 0 ≤ a ≤ q und b = 0 oder 1;
- Aryl oder Heteroaryl mit 2 bis 30 Kohlenstoffatomen und deren alkyl-, aryl-, heteroaryl-, cycloalkyl-, halogen-, hydroxy-, amino-, carboxy-, -O-, oder -CO-O- substituierte Komponenten, wie beispielsweise Phenyl, 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl, 4-Phenyl-phenyl, 1-Naphthyl, 2-Naphthyl, 1-Pyrrolyl, 2-Pyrrolyl, 3-Pyrrolyl, 2-Pyridinyl, 3-Pyridinyl, 4-Pyridinyl oder C₆F₍₅₋ₐ₎Hₐ mit 0 ≤ a ≤ 5; oder
- zwei Reste einen ungesättigten, gesättigten oder aromatischen, gegebenenfalls durch funktionelle Gruppen, Aryl, Alkyl, Aryloxy, Alkyloxy, Halogen, Heteroatome und / oder Heterocyclen substituierten und gegebenenfalls durch ein oder mehrere Sauerstoff- und/oder Schwefelatome und/oder ein oder mehrere substituierte oder unsubstituierte Iminogruppen unterbrochenen Ring.

In den vorstehenden Anionen bedeuten R^{a}, R^{b}, R^{c} und R^{d} bevorzugt unabhängig voneinander jeweils ein Wasserstoffatom oder eine C₁-C₁₂-Alkylgruppe.

Beispielhaft seien folgende Anionen genannt: Chlorid; Bromid; Iodid; Thiocyanat; Hexafluorophosphat; Trifluormethansulfonat (Triflat); Methansulfonat; die Carboxylate, insbesondere Formiat; Acetat; Mandelat; Nitrat; Nitrit; Trifluoracetat; Sulfat; Hydrogensulfat; Methylsulfat; Ethylsulfat; 1-Propylsulfat; 1-Butylsulfat; 1-Hexylsulfat; 1-Octylsulfat; Phosphat; Dihydrogenphosphat; Hydrogenphosphat; C₁-C₄-Dialkylphosphate; Propionat; Tetrachloroaluminat; Al₂Cl₇⁻; Chlorozinkat; Chloroferrat; Bis(trifluoromethylsulfonyl)imid; Bis(pentafluoroethyl-sulfonyl)imid; Bis(nonafluorobutylsulfonyl)imid, Bis(heptadecafluorooctylsulfonyl)imid, Bis(methylsulfonyl)imid; Bis(p-Tolylsulfonyl)imid; Tris(trifluoromethylsulfonyl)methanid; Bis(pentafluoroethylsulfonyl)methanid; p-Tolylsulfonat; Tetracarbonylcobaltat; Dimethylenglykolmonomethylethersulfat; Oleat; Stearat; Acrylat; Methacrylat; Maleinat; Hydrogencitrat; Vinylphosphonat; Bis(pentafluoroethyl)phosphinat; Borate wie Bis[Salicylato(2-)]borat, Bis[oxalato(2-)]borat, Bis[1,2-benzoldiolato(2-)-O,O']borat, Tetracyanoborat, Tetrafluoroborat; Dicyanamid; Tris(pentafluoroethyl)trifluorophosphat; Tris(heptafluoropropyl)trifluorophosphat, Hexafluorophosphat, cyclische Arylphosphate wie Brenzcatecholphosphat (C₆H₄O₂)P(O)O- und Chlorocobaltat.

Bevorzugte Anionen sind solche aus der Gruppe
- der Alkylsulfate R^{a}OSO₃⁻, wobei R^{a} für eine C₁-C₁₂ Alkylgruppe, vorzugsweise für eine C₁-C₆ Alkylgruppe steht,
- der Alkylsulfonate R^{a}SO₃⁻; wobei R^{a} für eine C₁-C₁₂ Alkylgruppe, vorzugsweise für eine C₁-C₆ Alkylgruppe steht,
- der Arylsulfonate R^{a}SO₃⁻; wobei R^{a} für eine Aromaten steht, der mit einer C₁-C₁₂ Alkylgruppe substituiert ist, vorzugsweise mit einer C₁-C₆ Alkylgruppe substituiert ist,
- der perfluorierten Sulfonate R^{a}SO₃⁻; wobei R^{a} eine C₁-C₁₂ Alkylgruppe ist, die perfluoriert ist, vorzugsweise eine C₁-C₆ Alkylgruppe, die perfluoriert ist,
- der Halogenide, insbesondere Chlorid und Bromid und
- der Pseudohalogenide, wie Thiocyanat, Dicyanamid,
- der Carboxylate R^{a}COO-; wobei R^{a} für eine C₁-C₂₀ Alkylgruppe, vorzugsweise für eine C₁-C₈ Alkylgruppe steht, insbesondere Acetat,
- der Phosphate, insbesondere der Dialkylphosphate der Formel R^{a}R^{b}PO₄⁻, wobei R^{a} und R^{b} unabhängig voneinander für eine C₁ bis C₆ Alkylgruppe stehen; insbesondere stehen R^{a} und R^{b} für die gleiche Alkylgruppe, genannt seien Dimethylphosphat und Diethylphosphat,
- perfluorierte Phosphate wie beispielsweise Tris(pentafluoroethyl)trifluorophosphat; Tris(heptafluoropropyl)trifluorophosphat, Hexafluorophosphat
- der Phosphonate, insbesondere der Monoalkylphosphonsäureester der Formel R^{a}R^{b}PO₃⁻, wobei R^{a} und R^{b} unabhängig voneinander für eine C₁-C₆ Alkylgruppe stehen,
- der perfluorierten Phosphinate, insbesondere der perfluorierten Monoalkylphosphinsäureester der Formel R^{a}R^{b}PO₂⁻, wobei R^{a} und R^{b} unabhängig voneinander für eine C₁-C₆ Alkylgruppe stehen wie z.B. Bis(pentafluoroethyl)phosphinat
- der perfluorierten Sulfonylimide, wie Bis(trifluoromethylsulfonyl)imid; Bis(pentafluoroethylsulfonyl)imid; Bis(nonafluorobutylsulfonyl)imid, Bis(heptadecafluorooctylsulfonyl)imid
- der Methanide, wie z.B. Tricyanomethanid
- der perfluorierten Sulfonylmethanide, wie z.B. Tris(trifluoromethylsulfonyl)methanid, Bis(pentafluoroethylsulfonyl)methanid
- BF₄

Besonders bevorzugte Anionen sind Chlorid, Bromid, Hydrogensulfat, Tetrachloroaluminat, Thiocyanat, Dicyanamid, Tricyanomethanid, Methylsulfat, Ethylsulfat, Methansulfonat, Trifluormethansulfonat, Nonafluorobutansulfonat, Formiat, Acetat, Dimethylphosphat, Diethylphosphat, p-Tolylsulfonat, Tetrafluoroborat und Hexafluorophosphat, Methylmethanphosphonat, Methylphosphonat, und Bis(trifluoromethylsulfonyl)imid.

Oben genannte Kationen und Anionen verbinden sich zu Salzen, die bevorzugte Ionische Flüssigkeiten sind. Besonders bevorzugt sind Imidazoliumsalze der nachstehenden Formel II, wobei das Kation die in Formel I beschriebenen Eigenschaften aufweist: 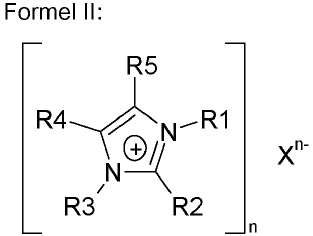 worin
R1 und R3 für einen unter Reaktionsbedingungen inerten organischen Rest mit 1 bis 20 C-Atomen stehen,
R2, R4, und R5 für ein H-Atom oder einen unter Reaktionsbedingungen inerten organischen Rest mit 1 bis 20 C-Atomen stehen,
X für ein Anion steht und
n für 1, 2 oder 3 steht.

In Formel II steht X für ein Anion, vorzugsweise einem der oben genannten Anionen.

Der Fachmann unterteilt die Stoffklasse der ionischen Flüssigkeiten auch nach ihrer Wassermischbarkeit in hydrophobe und hydrophile ionische Flüssigkeiten, die in der Regel auch schwach bis stark hygroskopisch sind. Eine Mischungslücke mit Wasser ermöglicht eine zweiphasige Reaktionen in Gegenwart von Wasser. Die Löslichkeit von Wasser in einer hydrophoben ionischen Flüssigkeit beträgt (bei Normalbedingungen, d.h. 20°C, 1 bar) weniger als 100g Wasser in 1000g ionischer Flüssigkeit, bevorzugt weniger als 50g, besonders bevorzugt weniger als 25g, insbesondere weniger als 10g. Der hydrophobe Charakter der ionischen Flüssigkeiten kann z.B. durch Halogenierung, im speziellen durch einen hohen Fluorierungsgrad, beispielsweise Perfluorierung des Anions erreicht.

Ganz besonders bevorzugte Anionen in Formel II sind demgemäß solche, die die Ionische Flüssigkeit hydrophob machen wie beispielsweise perfluorierte Sulfonate (z.B. C₄F₉SO₃⁻, C₈F₁₇SO₃⁻), perfluorierte Sulfonylimide (z.B. Bis(trifluoromethylsulfonyl)imid, Bis(pentafluoroethylsulfonyl)imid, Bis(nonafluorobutylsulfonyl)imid, Bis(heptadecafluorooctylsulfonyl)imid), perfluorierte Phosphinate (z.B. Bis(pentafluoroethyl)phosphinat), perfluorierte Phosphate (z.B. Tris(pentafluoroethyl)trifluorophosphat; Tris(heptafluoropropyl)trifluorophosphat, Hexafluorophosphat). Insbesondere bevorzugt ist Bis(trifluoromethylsulfonyl)imid.

In Formel II steht n vorzugsweise für 1.

Besonders bevorzugte Ionische Flüssigkeiten bestehen ausschließlich aus einem organischen Kation mit einem der vorstehenden Anionen.

Das Molgewicht der Ionischen Flüssigkeiten ist im allgemeinen kleiner 2000 g/mol, besonders bevorzugt kleiner 1500 g/mol, besonders bevorzugt kleiner 1000 g/mol und ganz besonders bevorzugt kleiner 750 g/mol.

Ionische Flüssigkeiten können auf Trägermaterialien aufgebracht werden. Die Bindung an eine Trägeroberfläche kann erfolgen durch:
- kovalente Bindung zwischen Silanol-Gruppen und dem Anion oder Kation der Ionischen Flüssigkeit
- nicht-kovalente Bindung in Form von Physisorption über van der Waals und Dipol Kräften.

Erfindungsgemäß werden solche Katalysatoren eingesetzt, bei denen Ionische Flüssigkeiten nicht-kovalent an einen Träger gebunden sind.

Als Träger kommen beispielsweise Aluminiumoxid, Titandioxid, Zirkondioxid, Siliziumdioxid, Magnesiumdioxid, einzeln oder in Kombination (z.B. Silizium- / Aluminiumoxid, Silizium- / Titandioxid, Silizium- / Zirkondioxid, Magnesium- / Aluminiumoxid, Titan- / Zirkondioxid) oder auch Zeolithe, Aktivkohlen oder Polymere in Betracht. Die Träger können in allen bekannten Modifikationen verwendet werden, beispielsweise können Katalysatoren auf Basis von γ-Al₂O₃, θ-Al₂O₃ oder α-Al₂O₃, auf Basis von monoklinem, tetragonalem oder kubischem ZrO₂, auf Basis von TiO₂ in Rutil- oder Anatasmodifikation hergestellt werden.

Die Trägermaterialien können Poren aufweisen, z.B. mit einem Porenvolumen 0,05 bis 1,0 ml/g.

Die erfindungsgemäß verwendeten Trägerkatalysatoren können entweder alleine oder in Kombination mit einer weiteren katalytisch aktiven Substanz verwendet werden.

Als katalytisch aktive Substanzen können den Ionischen Flüssigkeiten Substanzen, insbesondere Säuren zugesetzt werden. Besonders bevorzugt werden Lewis-Säuren zugesetzt wie z.B. ein Aluminium-, Scandium-, Indium-, Zink-, Eisen- oder Kupferhalogenid, -trifluormethansulfonat oder -oxid. Der Gehalt an Lewissäure, der der Ionischen Flüssigkeit zugesetzt werden kann, beträgt vorzugsweise 0,1-50 Gew.-%, bevorzugt von 0,1-10 Gew.-%, besonders bevorzugt kleiner 5 Gew.-%, jeweils bezogen auf den gesamten Trägerkatalysator.

Die katalytisch aktive Phase kann neben der Ionischen Flüssigkeit auch weitere Lösungsmittel enthalten, um z.B. die Fließeigenschaften der katalytisch aktiven Phase zu steuern. Zu beachten ist bei der Wahl des Lösemittels, dass dieses nicht mit der Ionischen Flüssigkeit oder einer etwaig zusätzlich vorhandenen Lewis-Säure reagiert und so die katalytisch aktive Phase chemisch verändert. Das eingesetzte Lösungsmittel soll im erfindungsgemäßen Prozess unverändert in der katalytisch aktiven Phase erhalten bleiben. Bei der Wahl des Lösungsmittels sind deshalb die gewählten Reaktionsbedingungen zu berücksichtigen. Beispielsweise wird man die Reaktionstemperaturen so wählen, dass das Lösungsmittel im erfindungsgemäßen Prozess nicht durch Abdampfen verloren geht. Vorzugsweise kommen Lösungsmittel mit Siedepunkten in Betracht, die größer sind als die Reaktionstemperatur. Zum Einsatz können beispielsweise Polyethylenglykole z.B. PEG 400 oder PEG 600 kommen.

Die Herstellung der immobilisierten Ionische Flüssigkeits - Phase Katalysatoren erfolgt nach dem Fachmann bekannten Methoden (beispielsweise Topics in Catalysis (1991), 14(1-4), 139-44 oder Green Chemistry (2002), 4, 88-93). Insbesondere soll der Trägerkatalysator vor der Immobilisierung der Ionischen Flüssigkeit oder des Ionische Flüssigkeit-Lewissäure-Gemisches auf demselben, getrocknet werden. Die Herstellung des SILP-Katalysators erfolgt bevorzugt unter Schutzgasatmosphäre.

Der Gehalt an auf das Trägermaterial aufgebrachtem Ionische Flüssigkeits - Phase Katalysator beträgt vorzugsweise 10-50 Gew.-%, besonders bevorzugt von 10-25 Gew.-%, jeweils bezogen auf den gesamten Trägerkatalysator.

Die immobilisierten Ionische Flüssigkeits - Phase Katalysatoren können (in Abhängigkeit vom verwendeten Trägerkatalysator) in Form von Pulver oder bevorzugt in Form von Formkörpern wie Strängen, Split, Ringen, Hohlzylindern, Kugeln oder Tabletten beispielsweise mit einem Durchmesser von 0,1 bis 5 mm, bevorzugt 1 bis 3 mm eingesetzt werden. Dem Katalysator kann zur Herstellung von Formkörpern Bindemittel zugesetzt werden.

Nachfolgend wird die erfindungsgemäße Reaktion in der Gasphase unter Einsatz der immobilisierten Ionische Flüssigkeits - Phase Katalysatoren beschrieben, die insbesondere auch in Gegenwart von Wasser durchgeführt werden kann.

In der erfindungsgemäßen Reaktion werden Aldehyde oder Ketone an ein Alken elektrophil addiert, gefolgt von der Addition eines Nukleophils an das entstehende Intermediat, sodass ein Homoallylalkohol selektiv als Reaktionsprodukt gewonnen wird.

Erfindungsgemäß dienen unsubstituierte aliphatische Alkene als Ausgangsstoffe. Erfindungsgemäß dient ein geradkettiges oder verzweigtes C₃-C₆ Alken als Ausgangsstoff, wie z.B. Propen, Buten, Isobuten, Penten, oder Hexen.

Unter Aldehyd ist ein aliphatischer, oder ein aromatischer Aldehyd zu verstehen. Ein aliphatischer Aldehyd ist ein geradkettiger, verzweigter oder zyklischer Aldehyd, der bevorzugt 1 bis 20 C-Atome, besonders bevorzugt 1 bis 10 C-Atome, insbesondere 1 bis 4 C-Atome aufweist, wobei die Alkylketten unsubstituiert oder durch unter den Reaktionsbedingungen inerte Gruppen substituiert sein können. Solche inerten Gruppen sind beispielsweise Arylgruppen, wie Phenyl oder durch Alkyl, bevorzugt mit 1 bis 4 C-Atomen, wie Methyl, Ethyl, iso-Propyl, Butyl, iso-Butyl, durch Alkoxy, bevorzugt mit 1 bis 4 C-Atomen, wie Methoxy, Ethoxy, Propoxy, Butoxy oder durch Halogen substituiertes Phenyl.

Aromatische Aldehyde sind Aldehyde, bei denen eine oder mehrere Aldehydgruppen direkt an ein aromatisches C-Atom gebunden sind, beispielsweise in einer Phenyl-, Naphthyl-, oder Pyridylgruppe wie Benzaldehyd, Phthalaldehyd, Naphthylaldehyde, Pyridylaldehyd oderdialdehyd.

Unter Keton ist ein aliphatisches, oder ein aromatisches Keton zu verstehen. Ein aliphatisches Keton ist ein geradkettiges, verzweigtes oder zyklisches Keton, das bevorzugt 3 bis 20 C-Atome, besonders bevorzugt 3 bis 10 C-Atome aufweist, wobei die Alkylketten unsubstituiert oder durch unter den Reaktionsbedingungen inerte Gruppen substituiert sein können. Solche inerten Gruppen sind beispielsweise Arylgruppen, wie Phenyl oder durch Alkyl, bevorzugt mit 1 bis 4 C-Atomen, wie Methyl, Ethyl, iso-Propyl, Butyl, iso-Butyl, durch Alkoxy, bevorzugt mit 1 bis 4 C-Atomen, wie Methoxy, Ethoxy, Propoxy, Butoxy oder durch Halogen substituiertes Phenyl.

Aromatische Ketone sind Ketone, bei denen eine Ketogruppe direkt an mindestens ein aromatisches C-Atom gebunden ist, beispielsweise an eine Phenyl-, Naphthyl-, oder Pyridylgruppe. An der zweiten Bindungsstelle liegt dann ein aliphatisches C-Atom vor, das Alkyl, bevorzugt mit 1 bis 6, besonders bevorzugt mit 1 bis 4 C-Atomen, wie Methyl, Ethyl, iso-Propyl, Butyl, neoPentyl, Hexyl ist, z.b. Methylphenylketon. Die Ketogruppe kann auch an zwei aromatische C-Atome gebunden sein, wie z.B. Benzophenon.

Bevorzugt werden unsubstituierte, aliphatische Aldehyde mit 1 bis 10 C-Atomen eingesetzt. Besonders bevorzugt erfolgt die Umsetzung mit aliphatischen C₁-C₄ Aldehyden, wie beispielsweise Formaldehyd, Acetaldehyd, Propionaldehyd. Ganz besonders bevorzugt erfolgt die Umsetzung mit Formaldehyd.

Die Reaktion wird bevorzugt mit einem Überschuss an Alken durchgeführt. Der Überschuss kann dabei sehr groß sein (10-20-fach molarer Überschuss bezogen auf den eingesetzten Aldehyd oder das eingesetzte Keton). Bevorzugt beträgt der Überschuss das 15-fache bezogen auf den eingesetzten Aldehyd, besonders bevorzugt das 10-fache, ganz besonders bevorzugt weniger als das 5-fache. In einer besonderen Ausführungsform wird der unverbrauchte Überschuss wieder in das System rückgeführt.

Die Reaktion wird mithilfe von den oben beschriebenen Katalysatorensystemen durchgeführt. Dies ermöglicht die Ausführung im Besonderen unter Verwendung von wässrigem Formaldehyd.

Die Reaktionstemperatur liegt im Allgemeinen bei ≥ 50°C bis maximal 350°C. Bevorzugt sind Bereiche von 100-300°C, besonders bevorzugt von 100-250, ganz besonders bevorzugt von 100-200°C. Die Reaktionstemperatur wird so gewählt, dass sie im Bereich der thermischen Stabilität der Ionischen Flüssigkeit liegt, die abhängig von der Wahl der Ionischen Flüssigkeit ist und die der Fachmann beurteilen kann.

Die Reaktion kann im Unterdruck (<1 bar), im Normaldruck (=1 bar) oder im Überdruck (>1 bar) durchgeführt werden. Bevorzugt wird die Durchführung im Bereich des Normaldrucks.

Die Reaktionsführung kann diskontinuierlich oder kontinuierlich, vorzugsweise kontinuierlich erfolgen.

Die Isolierung der Reaktionsprodukte erfolgt in üblicher Weise, im Falle des Isoprenols z.B. über Kondensierung. Eine weitere Aufreinigung kann z.B. über Destillation erfolgen.

Beispiele:

### (1) Katalysatorherstellung:

### 3,1 g AlCl₃ (Lewissäure) wurden in 27,9 g EMIM TFSI (1-Ethyl-3-methylimidazolium

Bis(trifluoromethylsulfonyl)imide; Ionische Flüssigkeit) gelöst. Anschließend wurden 61,8 g D11-10® 3 mm Stränge (SiO₂; Trägermaterial) in 100 g Dichlormethan (organisches Lösungsmittel) suspendiert und mit der Mischung AlCl₃ / EMIM TFSI versetzt. Die Suspension wurde gerührt und anschließend das Lösungsmittel im Vakuum entfernt. Es blieben 91,3 g immobilisierter Ionische Flüssigkeits-Phase - Katalysator.

### (2) Gasphasenreaktion

48 g Katalysator (D11-10® Split beladen mit 50 Gew.-% EMIM TFSI und 10 Gew.-% AlCl₃ bezogen auf den gesamten Trägerkatalysator) wurden in die Gasphasenapperatur eingebaut. Bei 150°C Reaktionstemperatur wurden 10 g/h (0,12 mol/h) 36,5%ige Formaldehydlösung (rund 0,2 g/gKat/h), 5,4 Normliter/h (0,24 mol/h) Isobuten (gasförmig über eine Gasflasche zudosiert) und 2 Normliter/h N₂ (Trägergas) über die Apparatur gefahren. Nach 8 h Betriebszeit enthielt der Reaktionsaustrag (über einen Kühler und eine Kühlfalle bei 0°C kondensiert) bei einem Isobutenumsatz von 5% Isoprenol und Isopren in einem Verhältnis von 26,53:1,37 Flächen%-GC.

## Patentansprüche

1. Verfahren zur Herstellung von Homoallylalkoholen durch katalysierte Umsetzung von geradkettigen oder verzweigten C₃-C₆ Alkenen mit Aldehyden oder Ketonen, **dadurch gekennzeichnet, dass** die Umsetzung in Gegenwart von nicht-kovalent immobilisierten Ionische Flüssigkeits - Phase Katalysatoren in der Gasphase erfolgt, wobei die nicht-kovalent immobilisierten Ionische Flüssigkeits - Phase Katalysatoren hydrophobe ionische Flüssigkeiten sind, die ausgewählt sind unter Imidazoliumsalzen, welche nicht-kovalent an einen Träger gebunden sind und welche wenigstens eine Lewis-Säure umfassen,
wobei die Wasserlöslichkeit der hydrophoben ionischen Flüssigkeit bei 20°C und 1 bar weniger als 100 g Wasser in 1000 g ionischer Flüssigkeit beträgt.

2. Verfahren gemäß Anspruch 1, **dadurch gekennzeichnet, dass** man als Aldehyd wässrigen Formaldehyd einsetzt.

3. Verfahren gemäß einem der vorangegangenen Ansprüche, **dadurch gekennzeichnet, dass** der Katalysator ein perfluoriertes Anion enthält.

4. Verfahren gemäß einem der vorangegangenen Ansprüche, **dadurch gekennzeichnet, dass** man Isobuten mit wässrigem Formaldehyd umsetzt.

5. Verfahren gemäß einem der vorangegangenen Ansprüche, **dadurch gekennzeichnet, dass** man das Verfahren bei Temperaturen ≥ 50°C bis maximal 350°C durchführt.

6. Verfahren gemäß einem der vorangegangenen Ansprüche, **dadurch gekennzeichnet, dass** man das Alken im 10-20-fach molarem Überschuss bezogen auf den Aldehyd oder das Keton einsetzt.

## Claims

1. A process for preparing homoallyl alcohols by catalyzed reaction of straight-chain or branched C₃-C₆ alkenes with aldehydes or ketones, wherein the reaction is carried out in the gas phase in the presence of noncovalently supported ionic liquid phase catalysts, where the noncovalently supported ionic liquid phase catalysts are hydrophobic ionic liquids selected from among imidazolium salts which are noncovalently bound to a support and comprise at least one Lewis acid and the water solubility of the hydrophobic ionic liquid at 20°C and 1 bar is less than 100g of water in 1000g of ionic liquid.

2. The process according to claim 1, wherein aqueous formaldehyde is used as aldehyde.

3. The process according to either of the preceding claims, wherein the catalyst comprises a perfluorinated anion.

4. The process according to any of the preceding claims, wherein isobutene is reacted with aqueous formaldehyde.

5. The process according to any of the preceding claims, wherein the process is carried out at temperatures of from ≥ 50°C to not more than 350°C.

6. The process according to any of the preceding claims, wherein the alkene is used in a 10-20-fold molar excess based on the aldehyde or the ketone.

## Revendications

1. Procédé de fabrication d'alcools homoallyliques par réaction catalysée d'alcènes en C₃-C₆ linéaires ou ramifiés avec des aldéhydes ou des cétones, **caractérisé en ce que** la réaction a lieu dans la phase gazeuse en présence de catalyseurs en phase liquide ionique immobilisés par des liaisons non covalentes, les catalyseurs en phase liquide ionique immobilisés par des liaisons non covalentes étant des liquides ioniques hydrophobes qui sont choisis parmi les sels d'imidazolium, qui sont reliés par des liaisons non covalentes à un support et qui comprennent au moins un acide de Lewis,
la solubilité dans l'eau du liquide ionique hydrophobe à 20 °C et 1 bar étant inférieure à 100 g d'eau dans 1 000 g de liquide ionique.

2. Procédé selon la revendication 1, **caractérisé en ce que** du formaldéhyde aqueux est utilisé en tant qu'aldéhyde.

3. Procédé selon l'une quelconque des revendications précédentes, **caractérisé en ce que** le catalyseur contient un anion perfluoré.

4. Procédé selon l'une quelconque des revendications précédentes, **caractérisé en ce que** de l'isobutène est mis en réaction avec du formaldéhyde aqueux.

5. Procédé selon l'une quelconque des revendications précédentes, **caractérisé en ce que** le procédé est réalisé à des températures de ≥ 50 °C à au plus 350 °C.

6. Procédé selon l'une quelconque des revendications précédentes, **caractérisé en ce que** l'alcène est utilisé en un excès molaire d'un facteur de 10 à 20 par rapport à l'aldéhyde ou la cétone.
